# EUROPÄISCHE PATENTANMELDUNG

(11) **EP 2 374 496 A1**
(43) Veröffentlichungstag der Anmeldung: **12.10.2011**
(21) Anmeldenummer: 11005433.5
(22) Anmeldetag: 25.09.2008
(51) Int. Cl.: A61M 35/00, B05B 11/00, B05C 17/00

(54) **Medizinprodukt zum Applizieren eines flüssigen, medizinischen Behandlungsmediums auf einen zu behandelnden Bereich**

(30) Priorität: 27.09.2007 DE 102007046600
(62) Teilanmeldung aus: 08834535.0
(71) Anmelder: Linhardt GmbH & Co. KG, 94234 Viechtach (DE); Temmler Pharma GmbH & Co. KG, 35039 Marburg (DE)
(72) Erfinder: Sollinger, Horst, D-83620 Feldkirchen (DE); Beil, Johann, D-94234 (DE)
(74) Vertreter: Graf Glück Habersack Kritzenberger

(57) **Zusammenfassung**

Stiftartiger Applikator (1,1a) zum gezielten Applizieren eines flüssigen medizinischen Behandlungsmediums bei der äußeren Behandlung eines menschlichen oder tierischen Körpers an einem zu behandelnden Bereich.

## Beschreibung

Die Erfindung bezieht sich auf ein Verfahren gemäß Oberbegriff

### Patentanspruch 1.

Aufgabe der Erfindung ist es, ein Verfahren sowie ein Medizinprodukt zu schaffen, mit dem ein besonders einfaches, gut dosiertes und vor allem auch genau lokalisiertes Aufbringen eines flüssigen medizinischen Behandlungsmediums auf einen zu behandelnden Bereich (Haut- oder Gewebebereich) eines menschlichen und/oder tierischen Körpers möglich ist. Zur Lösung dieser Aufgabe ist ein Verfahren entsprechend dem Patentanspruch 1 ausgebildet. Ein Medizinprodukt ist Gegenstand des Patentanspruchs 28.

Unter "medizinisches Behandlungsmedium" ist im Sinne der Erfindung ein Medium zu verstehen, welches in Lösung wenigstens einen medizinischen Wirkstoff enthält, beispielsweise zur Desinfektion, zur Behandlung von chronischen Wunden, wie z.B. Ulcus cruris oder Decubitus, sowie auch für andere medizinische Indikationen, beispielsweise zur Behandlung von Hornhaut, Warzen, Akne, Herpes, Onychomykosen, Hauptpilzerkrankungen, Pigmentveränderungen oder krankhafte Proliferationen der Haut- und Schleimhaut, und zwar jeweils durch äußere Anwendung.

Die den wenigstens einen Wirkstoff enthaltende Lösung ist beispielsweise eine ölige Lösung oder eine wässrige, alkoholische Lösung und dabei so ausgewählt, dass der erforderliche Fluss des Behandlungsmediums aus dem röhrchen- oder hülsenförmigen Behälter durch die Applikationsspitze an deren zum Applizieren dienendes Ende (Applikationsende) insbesondere auch ohne ein Zusetzen der Poren und/oder Kanäle innerhalb der Applikationsspitze gewährleistet ist, die Lösung aber auch die erforderliche Stabilität des Behandlungsmediums sicherstellt, insbesondere auch in physikalischer und/oder chemischer Hinsicht. Der verwendete Applikator weist beispielsweise die Größe eines üblichen Filzstiftes oder Markers auf. Es ist aber auch möglich, die Größe des Applikators und/oder das Volumen des zur Aufnahme des Behandlungsmediums dienenden Behälterinnenraumes oder Reservoirs dieses Applikators so klein zu wählen, dass das Behandlungsmedium lediglich für eine einmalige Behandlung oder aber nur für eine sehr begrenzte Anzahl von Behandlungen ausreichend ist.

Grundsätzlich erfolgt das Applizieren des Behandlungsmediums durch Bestreichen des zu behandelnden Bereichs mit der Applikationsspitze bzw. mit deren Applikationsende, wobei die Applikationsspitze bevorzugt aus einem für einen gesteuerten und dosierten Durchtritt des Behandlungsmediums geeigneten Material besteht, beispielsweise aus einem porösen Material, auch gesinterten Material, z.B. aus einem gesinterten Kunststoff.

In Weiterbildung der Erfindung sind das erfindungsgemäße Verfahren und/oder das erfindungsgemäße Medizinprodukt beispielsweise so ausgebildet,
dass das Behandlungsmedium wenigstens einen Wirkstoff in einer öligen Lösung enthält,
und/oder
dass die ölige Lösung des Behandlungsmediums mindestens ein mineralisches Öl und/oder wenigstens ein Derivat eines mineralischen Öls enthäl,
und/oder
dass das mineralische Öl Paraffinöl und/oder Silikonöl ist,
und/oder
dass das Behandlungsmedium oder dessen Lösung wenigstens ein pflanzliches Öl und/oder Ölprodukt enthält, welches aus wenigstens einem pflanzlichen Öl durch Umestern, Teilreduktion, Verethern und/oder Teilsynthese gewonnen ist, und/oder
dass als pflanzliches Öl Sonnenblumenöl und/oder andere in den Arzneibüchern monographierte, pflanzliche Öle verwendet werden,
und/oder
dass als Ölprodukt Glycerolester oder andere Ester von Festsäuren mit Fettalkoholen, Fettalkohole und/oder deren Ether und Ester verwendet werden, auch in beliebiger Mischung und in beliebigen Anteilen,
und/oder
dass die ölige Lösung zumindest einen Zusatz in Form von Tensiden, vorzugsweise mit einem Anteil von etwa 0,1 - 4,0 Gewichts-%, bezogen auf die Gesamtmasse der Lösung, und/oder in Form von Wachsen, vorzugsweise mit einem Anteil von etwa 0,1 - 1,0 Gewichts-% bezogen auf die Gesamtmasse der Lösung, und/oder Parfümöle und/oder Farbstoffe enthält,
und/oder
dass das Behandlungsmedium den wenigstens einen medizinischen Wirkstoff in einer wässrigen, alkoholischen Lösung enthält,
und/oder
dass die wässrige alkoholische Lösung ein- oder mehrwertige Alkohole, wie z.B. Ethanol, Glycerol und/oder polymere Alkohole und/oder Isopropanol und/oder Propylenglykol und/oder PEG und/oder deren Ester und/oder Ether enthält, und/oder
dass die wässrige Lösung Ethanol und/oder Isopropanol in einer Konzentration von etwa 5,0 - 70 Gewichts-%, bezogen auf die Gesamtmasse der Lösung enthält,
und/oder
dass die wässrige alkoholische Lösung die mehrwertigen, gesättigten und/oder ungesättigten Alkohole und polymeren Alkohole, wie z.B. Propylenglykol und/oder deren Ester und/oder Ether in einer Konzentration von etwa 2,0 - 40 Gewichts-%, bezogen auf die Gesamtmasse der Lösung enthält,
und/oder
dass die wässrige, alkoholische Lösung wenigstens einen medizinischen Wirkstoff in liposomaler und/oder nanosomaler Verkapselung enthält,
und/oder
dass die wässrige alkoholische Lösung einen Film bildenden Bestendteil, vorzugsweise einen Film bildenden polymeren Bestandteil enthält,
und/oder
dass als zusätzliches Lösungsmittel Ethylacetat, vorzugsweise in einer Konzentration von etwa 5,0 - 40 Gewichts-%, und/oder Aceton, vorzugsweise in einer Konzentration von etwa 5,0 - 40 Gewichts%, jeweils bezogen auf die Gesamtmasse der Lösung verwendet ist,
und/oder
dass als Film bildender Bestandteil Polymere und/oder Co- und Crosspolymere, wie z.B. Polymethacrylate, vorzugsweise auch chemisch modifiziert verwendet ist,
und/oder
dass die wässrige alkoholische Lösung als Zusatz Tenside, vorzugsweise in einer Konzentration von etwa 0,5 - 5 Gewichts-%, bezogen auf die Gesamtmasse der Lösung, und/oder Fettalkohole und/oder deren Ester und Ether, vorzugsweise in einer Konzentration von etwa 0,5 - 4,0 Gewichts%, bezogen auf die Gesamtmasse der Lösung enthält,
und/oder
dass Polysobat 80 und/oder Octyldodecanol als Zusatz verwendet ist,
und/oder
dass die wässrige alkoholische Lösung als Zusatz Verdicker auf Basis von Cellulose und/oder Stärke, beispielsweise Oligosaccharide und/oder Polyacrylate, vorzugsweise in einer Konzentration von etwa 0,1 - 0,4 Gewichts-%, bezogen auf die Gesamtmasse der Lösung enthält,
und/oder
dass die Lösung Parfümöle und/oder Farbstoffe enthält,
und/oder
dass wenigstens ein medizinischer Wirkstoff zur Behandlung von Hornhaut, Warzen, Akne, Herpes, Onychomykosen, Hauptpilzerkrankungen, Pigmentveränderungen oder krankhafte Proliferationen der Haut enthalten ist, und/oder
dass wenigstens ein medizinischer Wirkstoff zur Behandlung von chronischen Wunden, wie z.B. Ulcus cruris oder Decubitus enthalten ist,
und/oder
dass das Behandlungsmedium in der Lösung wenigstens einen enzymatischen und/oder antimikrobiellen Wirkstoff enthält,
und/oder
dass die Applikationsspitze aus einem porösen Material besteht, beispielsweise aus einem porösen gesinterten Material, z.B. aus einem gesinterten polymeren Material,
und/oder
dass die Applikationsspitze an ihrem zum Aufbringen des Behandlungsmediums auf die Haut dienenden Ende mit reduzierter Härte ausgebildet ist,
und/oder
dass die Applikationsspitze an ihrem zum Aufbringen des Behandlungsmediums dienenden Ende (5.1) eine reduzierte Porosität aufweist,
und/oder
dass die Applikationsspitze aus PE, PP, PVDF, PTFE und/oder EVA besteht, und/oder
dass der Behälter hülsenartig oder röhrchenförmig ausgebildet ist,
wobei die vorgenannten Merkmale jeweils einzeln oder in beliebiger Kombination verwendet sein können.

Weiterbildungen, Vorteile und Anwendungsmöglichkeiten der Erfindung ergeben sich auch aus der nachfolgenden Beschreibung von Ausführungsbeispielen und aus den Figuren. Dabei sind alle beschriebenen und/oder bildlich dargestellten Merkmale für sich oder in beliebiger Kombination grundsätzlich Gegenstand der Erfindung, unabhängig von ihrer Zusammenfassung in den Ansprüchen oder deren Rückbeziehung. Auch wird der Inhalt der Ansprüche zu einem Bestandteil der Beschreibung gemacht.

Die Erfindung wird im Folgenden anhand der Figuren an Ausführungsbeispielen näher erläutert. Es zeigen:
Fig. 1 in einer Explosionsdarstellung die Elemente einer Vorrichtung zum Applizieren eines flüssigen medizinisch wirksamen Behandlungsmediums gemäß der Erfindung;
Fig. 2 die Vorrichtung der Figur 1 im zusammengebauten Zustand;
Fig. 3 u. 4 Darstellungen wie Figuren 1 und 2 bei einer weiteren Ausführungsform.

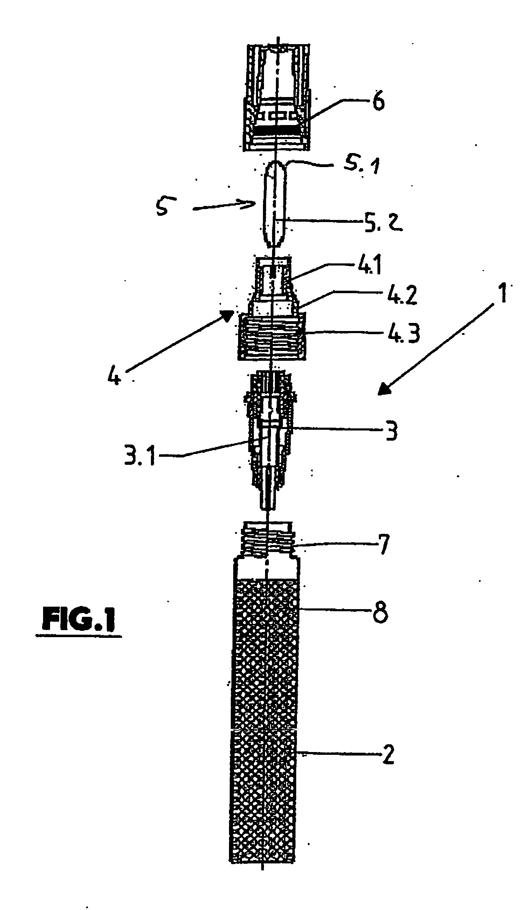

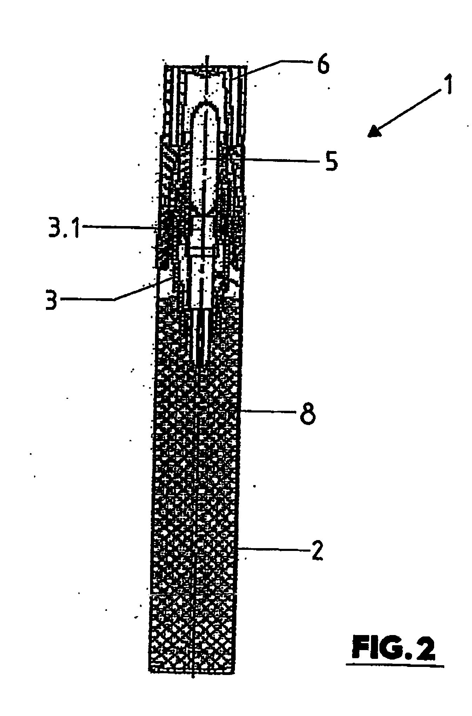

Die in den Figuren 1 und 2 allgemein mit 1 bezeichnete Vorrichtung (Applikator) besteht u.a. aus einem aus einem geeigneten Material, beispielsweise aus Kunststoff, Glas und/oder Metall hergestellten hülsen- oder röhrchenartigen, nur an einem Ende, d.h. bei der Darstellung der Figur 1 an seinem oberen Ende offenen Behälter 2 zur Aufnahme des Behandlungsmediums, aus einem eine Ventil- oder Dosierpumpe 3.1 enthaltenden Einsatz 3, aus einem hülsenartigen Mundstück 4 mit Abschnitten 4.1, 4.2 und 4.3 mit unterschiedlichem Innen- und Außendurchmesser aus einer Applikationsspitze 5 sowie aus einer Verschlusskappe 6, die ebenso wie zumindest Teile des Einsatzes 3 und des Mundstücks 4 als Formteil (Spritzgießteil) aus Kunststoff hergestellt ist.

Die Applikationsspitze 5 besteht aus einem porösen, Flüssigkeit leitenden Material und ist z.B. so ausgebildet, dass sie an ihrem im zusammengebauten Zustand des Applikators 1 aus dem Mundstück 4 vorstehenden Ende, d.h. an dem dortigen zum Applizieren des Behandlungsmediums dienenden Ende 5.1 (Applikationsende) weicher ausgeführt ist, als an ihrem übrigen Bereich bzw. Schaft 5.2. Weiterhin sitzt die Applikationsspitze 5 im Bereich ihres Endes 5.1 eine Porosität mit einer gegenüber dem Schaft 5.2 reduzierten Porengröße, d.h. die Porengröße am Ende 5.1 liegt beispielsweise im Bereich zwischen etwa 18 und 30 µm und im Bereich des Schaftes 5.2 im Bereich zwischen etwa 40 und 60 µm. Durch die gröbere Porenstruktur und die härtere Ausbildung der Applikationsspitze 5 am Schaft 5.2 wird u.a. die Zuführung des flüssigen Behandlungsmediums an das Ende 5.1 verbessert, gleichzeitig werden durch die härtere Ausbildung des Schaftes 5.1 die mechanischen Eigenschaften der Applikationsspitze an die Voraussetzungen einer maschinelle Montage wesentlich verbessert angepasst.

Als Material für die Applikationsspitze 5 eignen sich u.a. grundsätzlich poröse, Flüssigkeit leitende Materialien, beispielsweise poröse Kunststoffmaterialien, poröse Keramik, aber auch filz- oder fließartige Materialien. Bevorzugt besteht die Applikationsspitze aus einem gesinterten Material, z.B. aus einem gesinterten polymeren Material, z.B. PE, PP, PVDF, PTFE oder EVA. Wegen der hydrophoben Eigenschaften der polymeren Materialien ist es zweckmäßig, diese Materialien hydrophil zu behandeln.

Weiterhin kann es zweckmäßig sein, die Applikationsspitze 5 an ihrer Umfangsfläche mit wenigstens einer sich über einen Großteil der Länge oder über die gesamte Länge der Applikationsspitze 5 erstreckenden Be- oder Entlüftungsnut zu versehen, um ein insbesondere auch thermisch bedingtes Tropfen oder Auslaufen des Applikators 1 zu vermeiden. Die Applikationsspitze 5 ist weiterhin bevorzugt zumindest an ihrem Ende 5.1, beispielsweise an beiden Enden sich verjüngend ausgebildet, und zwar dann z.B. mit abgerundeter Spitze.

Im zusammengebauten Zustand ist der Einsatz 3 in die Öffnung des Behälters 2 eingesetzt und dort in geeigneter Weise, z.B. durch Verschrauben des mit einem Innengewinde versehenen Abschnittes 4.3 des Mundstückes 4 gesichert, sodass der Einsatz 3 an der Öffnung des Behälters 2 diese Öffnung abdichtend gehalten ist, mit einem röhrchenförmigen Einlass seiner Dosierpumpe 3.1 in den Innenraum des Behälters 2 bzw. in das dort aufgenommene flüssige Behandlungsmedium 8 hineinreicht und mit dem Auslass seiner Dosierpumpe 3.1 im Innenraum des Abschnittes 4.2 des Mundstückes 4 endet. Die Applikationsspitze 5 ist mit ihrem Schaft 5.2 in das Mundstück 4 eingesetzt, und zwar derart, dass sie mit ihrem Ende 5.1 über das in den Figuren 1 und 2 obere Ende des Mundstückes 4 vorsteht und mit ihrem unteren Ende in den Innenraum des Abschnittes 4.2 bzw. in das dort aufgenommene obere Ende des Einsatzes 3 hineinreicht und der Dosierpumpe axial unmittelbar gegenüberliegt. Durch axiales Verschieben der Applikationsspitze 5 gegen die Wirkung einer nicht dargestellten, beispielsweise in der Dosierpumpe 3.1 vorgesehenen Rückstellfeder kann die Dosierpumpe 3.1 betätigt werden, und zwar zur Entnahme einer dosierten Menge des Behandlungsmediums 8 aus dem Behälter 2 und zur Abgabe dieser Menge an die Applikationsspitze 5.

Es versteht sich, dass die Dosierpumpe 3.1 auch andersartig ausgebildet sein kann, und zwar so, dass sie durch Ausübung eines radialen mechanischen Druckes auf das Mundstück 4 und/oder auf den beispielsweise elastisch verformbaren Behälter 2 betätigt wird. Weiterhin ist es auch möglich, anstelle der Dosierpumpe 3.1 ein Dosierventil vorzusehen, welches bei Ausübung des radialen Druckes auf den Applikator 1, insbesondere auf das Mundstück und/oder auf den elastisch ausgebildeten Behälter 2 für die Abgabe einer dosierten Menge des Behandlungsmediums aus dem Behälter öffnet.

Bei Nichtgebrauch der Vorrichtung ist auf das Mundstück 4 die Verschlusskappe 6 aufgesetzt, in der dann auch das über das Mundstück 4 vorstehende Ende 5.1 der Applikationsspitze 5 dicht verschlossen aufgenommen ist.

Zum Applizieren des Behandlungsmediums 8 auf dem zu behandelnden Bereich, z.B. auf die zu behandelnde Haut- oder Gewebepartie wird der zu behandelnden Bereich nach Abnahme der Verschlusskappe 6 und gegebenenfalls nach Betätigung der Dosierpumpe 3.1 oder eines entsprechenden Dosierventils mit dem Ende 5.1 der Applikationsspitze 5 bestrichen. Durch die Dosierpumpe 3.1 bzw. durch das entsprechende Dosierventil wird hierbei nicht nur die dosierte Abgabe des Behandlungsmediums 8 aus dem Innenraum des Behälters 2 erreicht, sondern insbesondere auch verhindert, das Behandlungsmedium von der Applikationsspitze 5 zurück in den Innenraum des Behälters 2 gelangt. Hierdurch wird die Haltbarkeit des Behandlungsmediums 8 im Behälter 2 wesentlich verbessert, vor allem auch eine Verkeimung des im Behälter 2 aufgenommenen Behandlungsmediums 8 vermieden. Mit dem Applikator 1 ist ein unproblematisches, gut dosiertes und auch exakt lokalisiertes Aufbringen des flüssigen Behandlungsmediums 8 auf den zu behandelnden Bereich möglich. Der erfindungsgemäße Applikator ist also insbesondere, aber nicht ausschließlich überall dort bevorzugt verwendbar, wo eine medizinische Behandlung mit einem flüssigen Behandlungsmedium insbesondere auch lokal begrenzt erfolgen soll.

Der Applikator 1 eignet sich beispielsweise zur Behandlung von chronischen Wunden, wie Ulcus cruris oder Decubitus, wobei in diesen Fällen das Behandlungsmedium z. B. enzymatische und/oder antimikrobielle Wirkstoffe in einer geeigneten Lösung enthält und bei der Applikation eine exakte Abgrenzung des behandelten Bereichs zum angrenzenden Gewebe bzw. zur umgebenden Haut zumindest sehr vorteilhaft ist, um toxische Störungen in der Umgebung einer Wunde zu vermeiden, die die Haut für Mazeration anfällig machen.

Der Applikator 1 eignet sich weiter hin zum Aufbringen eines Behandlungsmediums auf kleine Wunden und Narben, insbesondere auch für alle externen Anwendungen eines medizinischen Wirkstoffes, der bei seiner Anwendung lokal sehr genau aufgebracht werden muss, insbesondere auch in unmittelbarer Nähe kritischer Bereiche, wie z.B. Augen, Schleimhäute usw.

Der Applikator 1 eignet sich weiter zum Auftragen von Lösungen auf die Haut oder den Nagel mit Wirkstoffen zur Behandlung von Haut- und Nagelmykosen und deren Begleiterscheinungen.

Der Applikator 1 eignet sich auch zur Aufbringung von wirkstoffhaltigen Lösungen zur Behandlung von Warzen, Verhornungen sowie bakteriellen und viralen Hauterkrankungen.

Im Detail eignet sich der Applikator 1 mit einem entsprechenden, im Behälter 2 aufgenommenen medizinischen Behandlungsmedium zur Behandlung von Hornhaut, Warzen, Akne, Herpes, Onychomykosen, Pilzerkrankungen, Pigmentveränderungen, krankhafte Proliferationen der Haupt usw.

Von besonderer Bedeutung für die Funktionstüchtigkeit des Applikators 1 und das Behandlungsergebnis, aber auch für die Stabilität des Behandlungsmediums ist die verwendete Lösung, in der der für die jeweilige medizinische Indikation erforderliche Wirkstoff aufgenommen ist.

Als Lösung eignen sich beispielsweise ölige Lösungen, die dann z.B.

mineralische Öle enthalten, wie Paraffinöle, Silikonöle und/oder deren Derivate,

pflanzliche Öle, wie z.B. Sonnenblumenöl,

aus pflanzlichen Ölen durch Umestern und/oder Teilsynthesen gewonnene Ölprodukte, wie Glycerolester oder andere Ester von Fettsäuren, auch mit Fettalkoholen, Fettalkohole, deren Ether und/oder Ester, enthalten,

und zwar jeweils als Einzelbestandteil und/oder in beliebiger Mischung und/oder in unterschiedlichsten Einsatzkonzentrationen.

Derartige Lösungen können weiterhin Zusätze enthalten, wie z.B. Tenside und/oder Wachse, letztere zur Verdickung der Ölphase, Parfümöle und/oder Farbstoffe. Die Tenside sind dann beispielsweise in einen Anteil von etwa 0,1 - 4,0 Gewichts-% und die Wachse in einem Anteil von 0,1 - 1,0 Gewichts-% in der Lösung enthalten, und zwar jeweils bezogen auf die Gesamtmasse der Lösung.

Als Lösung eignen sich weiterhin wässrige, alkoholische Lösungen. Diese enthalten Wasser (aqua dest.) und Alkohole, beispielsweise einwertige und/oder zweiwertige Alkohole wie z.B. Ethanol oder Isopropanol, und zwar z.B. in einer Konzentration von etwa 5 - 70 Gewichts-% bezogen auf das Gesamtgewicht der Lösung, und/oder polymere Ethylen/propylen-Glykole PEG, und/oder mehrwertige, gesättigte und ungesättigte Alkohole, wie z.B. Propylenglykol und/oder Ester und Ether von Alkoholen in einer Konzentration von z.B. 2 - 40 Gewichts-% bezogen auf die Gesamtmasse der jeweiligen Lösung.

Diese alkoholischen Lösungen können ebenfalls weitere Zusätze enthalten, insbesondere auch zur Verbesserung der Stabilität und/oder der physikalischen Eigenschaften und/oder Resorptionsverbesserung der jeweiligen Lösung und damit des Behandlungsmediums. Geeignete Zusätze sind z.B. Tenside zur Verbesserung der Löslichkeit und Oberflächenspreizung, wie z.B. Polysobat 80, bevorzugt in einer Konzentration von 0,5 - 5,0 Gewichts-%, Fettalkohole, deren Ester und Ether, wie z.B. Octyldodecanol, bevorzugt in einer Konzentration von etwa 0,5 - 4,0 Gewichts-%, Verdicker auf Basis von Zellulose, Stärke, Oligosaccharide und/oder Polyacrylate, bevorzugt in eine Konzentration von etwa 0,1 - 0,4 Gewichts-%, Parfümöle und/oder Farbstoffe. Die vorgenannten Angaben in Gewichts-% beziehen sich wiederum auf die Gesamtmasse der jeweiligen Lösung.

Speziell bei den wässrigen alkoholischen Lösungen besteht auch die Mögilchkeit, die Wirkstoffe, insbesondere auch die zur Behandlung der jeweiligen Indikation und dabei beispielsweise die zur Behandlung der vorstehend genannten Indikationen erforderlichen Wirkstoffe in liposomaler und/oder nanosomaler Verkapselung vorzusehen.

Geeignet sind weiterhin auch wässrige Lösungen, insbesondere wässrige alkoholische Lösungen der vorstehend genannten Art, die zusätzlich noch polymere Bestandteile zur Filmbildung, d.h. zur Ausbildung eines den jeweiligen Behandlungsbereich abdeckenden polymeren Filmes aufweisen. Diese Lösungen besitzen dann beispielsweise eine Zusammensetzung, wie sie vorstehend für die wässrigen alkoholischen Lösungen angegeben wurde, können aber auch noch andere Lösungsmittel, wie z.B. Ethylacetat vorzugsweise in einer Konzentration von etwa 5,0 - 40 Gewichts-%, Aceton, vorzugsweise in einer Konzentration von etwa 5,0 - 40 Gewichts-% und/oder Filmbrecher, wie z.B. andere Polymere und/oder Co- und Crosspolymere, wie z.B. Polymethacrylate (auch chemisch modifiziert), die geeignet sind, stabile, elastische Filme auf dem behandelten Bereich, beispielsweise auf der Haut, auf einem Nagel, auf krankhaft veränderten Gewebepartien, z.B. Warzen, Hornhaut usw. ausbilden, enthalten.

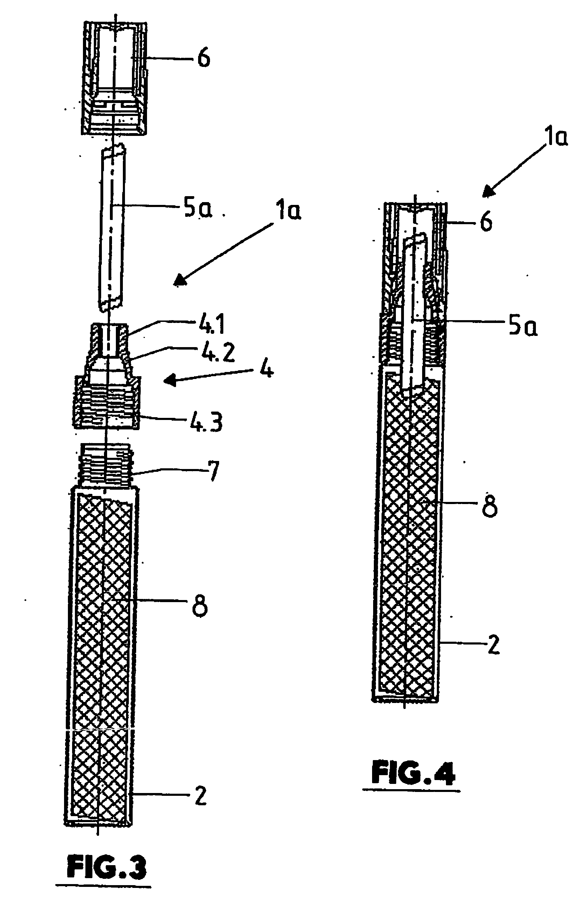

Die Figuren 3 und 4 zeigen eine Vorrichtung 1a, die sich von der Vorrichtung 1 im Wesentlichen nur dadurch unterscheidet, dass der Einsatz 3 mit der Dosierpumpe oder dem Dosierventil entfallen ist und dementsprechend die Applikationsspitze 5a unmittelbar in den Innenraum des Behälters bzw. in das dort aufgenommene Behandlungsmedium 8 hineinreicht. Ansonsten entspricht die Vorrichtung 1 a der Vorrichtung 1, sodass in den Figuren 3 und 4 für die der Vorrichtung 1 entsprechenden Elemente dieselben Bezugsziffern wie in den Figuren 1 und 2 verwendet sind. Auch die Vorrichtung 1a) eignet sich insbesondere für die Behandlung der vorstehend genannten Indikationen unter Verwendung eines Behandlungsmediums 8, welches den für die Behandlung der jeweiligen Indikation notwendigen Wirkstoff oder die entsprechende Wirkstoffkombination enthält, und zwar wiederum in einer öligen Lösung, einer wässrigen, alkoholischen Lösung und/oder einer wässrigen, bzw. wässrigen alkoholischen Lösung mit zusätzlichen polymeren Bestandteilen zur Filmbildung.

Die Erfindung wurde voranstehend an Ausführungsbeispielen beschrieben. Es versteht sich, dass zahlreiche Änderungen sowie Abwandlungen möglich sind, ohne das dadurch der der Erfindung zugrunde liegende Erfindungsgedanke verlassen wird.

So besteht beispielsweise die Möglichkeit, anstelle der Dosierpumpe 3.1 oder eines Dosierventils andere Dosierpumpen und/oder ventilartig wirkende Mittel zur dosierten Abgabe des Behandlungsmediums und/oder zur Verhinderung eines Rückflusses des Behandlungsmediums in den Innenraum des Behälters 2 und/oder zur Behinderung eines Tropfens des Behandlungsmediums vorzusehen. Weiterhin wurde vorstehend davon ausgegangen, dass der Behälter 2 ein röhrchenförmiger oder hülsenartiger Behälter ist. Auch andere Formen sind denkbar, insbesondere kann der zur Aufnahme des Behandlungsmediums dienende Behälter auch tubenartig ausgeführt sein. Um die Applikationsspitze 5 zumindest an der Außenfläche ihres aus dem Mundstück 4 vorstehenden Endes 5.1 möglichst steril zu halten, kann es zweckmäßig sein, in der Verschlusskappe Mittel zur Desinfektion vorzusehen, beispielsweise in Form wenigstens eines in der Kappe angeordneten, mit einem Desinfektionsmittel getränkten Kissens, in welches das Ende 5.1 beim Aufsetzen der Verschlusskappe 6 eintaucht.

### Bezugszeichenliste

- 1, 1a: Vorrichtung bzw. Applikator
- 2: Behälter
- 3: Einsatz
- 3.1: Dosierpumpe
- 4: Mundstück
- 4.1 - 4.3: Abschnitte des Mundstücks 4
- 5, 5a: Applikationsspitze
- 5.1: Applikationsende der Applikationsspitze
- 5.2: Schaft der Applikationsspitze
- 6: Verschlusskappe
- 7: Außengewinde
- 8: Behandlungsmedium oder Behandlungslösung mit medizinischem Wirkstoff

## Patentansprüche

1. Medizinprodukt mit einem Applikator zum gezielten Aufbringen bzw.
Applizieren eines flüssigen Behandlungsmediums mit einem medizinischen Wirkstoff bei einer äußeren Behandlung eines menschlichen oder tierischen Körpers an einem zu behandelnden Bereich, mit einem stiftartigen Applikators (1, 1a), der in einem Reservoir oder Behälter (2) das Behandlungsmedium enthält und der an einem Ende mit einer Applikationsspitze (5) versehen ist, über die das Behandlungsmedium aus dem Inneren des Behälters (2) durch Überstreichen des zu behandelnden Bereichs auf diesen aufgebracht werden kann, wobei der Behälter beispielsweise hülsenartig oder röhrchenförmig ausgebildet ist,
**dadurch gekennzeichnet,**
**dass** die Applikationsspitze (5) aus einem porösen Material besteht und an ihrem zum Aufbringen des Behandlungsmediums dienenden Ende (5.1) eine reduzierte Porosität aufweist.

2. Medizinprodukt nach Anspruch 1, **dadurch gekennzeichnet, dass** das Behandlungsmedium den wenigstens einen Wirkstoff in einer öligen Lösung enthält, wobei die ölige Lösung beispielsweise mindestens ein mineralisches Öl, z.B. Paraffinöl und/oder Silikonöl und/oder wenigstens ein Derivat eines mineralischen Öls und/oder wenigstens ein pflanzliches Öl und/oder Ölprodukt enthält, welches aus wenigstens einem pflanzlichen Öl durch Umestern und/oder Teilsynthese gewonnen ist.

3. Medizinprodukt nach Anspruch 2, **dadurch gekennzeichnet, dass** als Ölprodukt Glycerolester oder andere Ester von Festsäuren mit Fettalkoholen, Fettalkohole und/oder deren Ether und Ester verwendet ist, auch in beliebiger Mischung und in beliebigen Anteilen.

4. Medizinprodukt nach einem der Ansprüche 1 - 3, **dadurch gekennzeichnet, dass** die ölige Lösung zumindest einen Zusatz in Form von Tensiden, vorzugsweise mit einem Anteil von etwa 0,1 - 4,0 Gewichts-%, bezogen auf die Gesamtmasse der Lösung, und/oder in Form von Wachsen, vorzugsweise mit einem Anteil von etwa
0,1 - 1,0 Gewichts-% bezogen auf die Gesamtmasse der Lösung, und/oder Parfümöle und/oder Farbstoffe enthält.

5. Medizinprodukt nach einem der Ansprüche 1 - 4, **dadurch gekennzeichnet, dass** das Behandlungsmedium den wenigstens einen medizinischen Wirkstoff in einer wässrigen, alkoholischen Lösung enthält
wobei die wässrige alkoholische Lösung beispielsweise ein- oder mehrwertige Alkohole, wie z.B. Ethanol und/oder Isopropanol und/oder Propylenglykol und/oder deren Ester und/oder Ether und/oder Ethanol und/oder Isopropanol in einer Konzentration von etwa 5,0 - 70 Gewichts-%, bezogen auf die Gesamtmasse der Lösung enthält.

6. Medizinprodukt nach Anspruch 5, **dadurch gekennzeichnet, dass** die wässrige alkoholische Lösung die mehrwertigen, gesättigten und/oder ungesättigten Alkohole, wie z.B. Propylenglykol und/oder deren Ester und/oder Ether in einer Konzentration von etwa 2,0 - 40 Gewichts-%, bezogen auf die Gesamtmasse der Lüsung enthält,
und/oder
dass die wässrige alkoholische Lösung den wenigstens einen medizinischen Wirkstoff in lioposomaler und/oder nanosomaler Verkapselung enthält, und/oder
dass die wässrige alkoholische Lösung einen Film bildenden Bestendteil, vorzugsweise einen Film bildenden polymeren Bestandteil enthält,
wobei als Film bildender Bestandteil beispielsweise Ethylacetat, vorzugsweise in einer Konzentration von etwa 5,0 - 40 Gewichts-%, und/oder beispielsweise Aceton, vorzugsweise in einer Konzentration von etwa 5,0 - 40 Gewichts%, jeweils bezogen auf die Gesamtmasse der Lösung, und/oder Polymere und/oder Crosspolymere, wie z.B. Polymethacrylate, vorzugsweise auch chemisch modifiziert verwendet sind.

7. Medizinprodukt nach einem der Ansprüche 1 - 6, **dadurch gekennzeichnet, dass** die wässrige alkoholische Lösung als Zusatz Tenside, vorzugsweise in einer Konzentration von etwa 0,5 - 5 Gewichts-%, bezogen auf die Gesamtmasse der Lösung, und/oder Fettalkohole und/oder deren Ester und/oder Ether, vorzugsweise in einer Konzentration von etwa 0,5 - 4,0 Gewichts%, bezogen auf die Gesamtmasse der Lösung enthält,
wobei beispielsweise Polysobat 80 und/oder Octyldodecanol als Zusatz verwendet sind.

8. Medizinprodukt nach einem der Ansprüche 1 - 7, **dadurch gekennzeichnet, dass** die wässrige alkoholische Lösung als Zusatz Verdicker auf Basis von Cellulose und/oder Stärke, beispielsweise Oligosaccharide und/oder Polyacrylate, vorzugsweise in einer Konzentration von etwa 0,1 - 0,4 Gewichts-%, bezogen auf die Gesamtmasse der Lösung enthält.

9. Medizinprodukt nach einem der Ansprüche 1 - 8, **dadurch gekennzeichnet, dass** die Lösung Parfümole und/oder Farbstoffe enthält.

10. Medizinprodukt nach einem der Ansprüche 1 - 9, **gekennzeichnet durch** wenigstens einen medizinischen Wirkstoff zur Behandlung von Hornhaut, Warzen, Akne, Herpes, Onychomykosen, Hauptpilzerkrankungen, Pigmentveränderungen, krankhaften Proliferationen der Haut oder zur Behandlung von chronischen Wunden, wie z.B. Ulcus cruris oder Decubitus.

11. Medizinprodukt nach einem der Ansprüche 1 - 10, **dadurch gekennzeichnet, dass** das Behandlungsmedium in der Lösung wenigstens einen enzymatischen und/oder antimikrobiellen Wirkstoff enthält.

12. Medizinprodukt nach einem der Ansprüche 1 - 11, **gekennzeichnet durch** eine Applikationsspitze aus einem porösen gesinterten Material, z.B. aus einem gesinterten polymeren Material,
und/oder
**durch** eine Applikationsspitze (5), die an ihrem zum Aufbringen des Behandlungsmediums auf die Haut dienenden Ende mit reduzierter Härte ausgebildet ist,
und/oder
**durch** eine Applikationsspitze (5), die an ihrem zum Aufbringen des Behandlungsmediums dienenden Ende (5.1) eine reduzierte Porosität aufweist.

13. Medizinprodukt nach einem der Ansprüche 1 - 12, **dadurch gekennzeichnet, dass** die Applikationsspitze (5) aus PE, PP, PVDF, PTFE und/oder EVA besteht.
